# EUROPEAN PATENT APPLICATION

(11) **EP 2 223 646 A1**
(43) Date of publication of application: **01.09.2010**
(21) Application number: 08865416.5
(22) Date of filing: 03.12.2008
(51) Int. Cl.: A61B 1/00, A61B 1/04, G02B 23/24, G02B 23/26

(54) **BIOLOGICAL OBSERVATION APPARATUS AND ENDOSCOPIC APPARATUS**

(30) Priority: 25.12.2007 JP 2007333011
(71) Applicant: Olympus Corporation, Shibuya-ku Tokyo 151-0072 (JP)
(72) Inventor: FUJINUMA, Ken, Tokyo 151-0072 (JP); WATANABE, Takeshi, Tokyo 151-0072 (JP)
(74) Representative: Gunzelmann, Rainer
(86) International application number: PCT/JP2008/071980
(87) International publication number: WO 2009/081706

(57) **Abstract**

A blood vessel lying deep inside a biological tissue can be detected more clearly despite the existence of the biological tissue, particularly, a fat tissue. A biological observation apparatus (1) comprises: a light source unit (4) capable of selectively emitting illumination light beams of two kinds of wavelengths having different absorption intensities with respect to a substance contained in blood; an irradiation optical system (7) for irradiating a subject with the illumination light beams from the light source unit (4); a detection optical system (8, 9, 12) for detecting scattered light beams when the subject is irradiated with the illumination light beams of two kinds of wavelengths by the irradiation optical system (7), and acquiring respective images thereof; and an image processing section (14) for performing comparison operation processing on the two images acquired by the detection optical system (8, 9, 12).

## Description

### Technical Field

The present invention relates to a biological observation apparatus and an endoscopic apparatus.

### Background Art

Heretofore, there is a known imaging system for detecting the running pattern of blood vessels by irradiating a biological tissue with infrared light and capturing the image of the biological tissue (for example, refer to Patent Citation 1).

Patent Citation 1: Japanese Unexamined Patent Application, Publication No. 2004-358051

### Disclosure of Invention

However, if different types of biological substances are mixedly present within a same observation area, optical observation from the outside of the organism's body would be difficult. Particularly, light is strongly scattered by the surface of a biological tissue, for example a fat tissue. Therefore, the irradiated infrared light is scattered on the surface of the fat tissue, and thus the surface profile is enhanced in the acquired image, which problematically makes it difficult to detect the running pattern of a blood vessel lying deep inside the biological tissue.

The present invention was made to address such a situation, with an object of providing a biological observation apparatus and an endoscopic apparatus, with which a biological substance can be readily observed from the outside of an organism's body. Moreover, it is also an object of the present invention to provide a biological observation apparatus and an endoscopic apparatus, with which a blood vessel lying deep inside a biological tissue can be detected more clearly despite the existence of the biological tissue, particularly, a fat tissue.

In order to achieve the above objects, the present invention provides the following solutions.
The present invention provides a biological observation apparatus comprising: a light source unit capable of selectively emitting illumination light beams of two kinds of wavelengths having different absorption intensities with respect to a biological substance; an irradiation optical system for irradiating a subject with the illumination light beams from the light source unit; a detection optical system for detecting scattered light beams when the subject is irradiated with the illumination light beams of the two kinds of wavelengths by the irradiation optical system, and acquiring respective images thereof; and an image processing section for performing comparison operation processing on the two images acquired by the detection optical system.

According to the present invention, illumination light beams of two kinds of wavelengths are selectively emitted by the operation of the light source unit, and are irradiated on the subject by the irradiation optical system. The thus irradiated two kinds of illumination light beams are scattered by the subject, and are detected by the detection optical system, by which the respective images of these light beams are created. These illumination light beams have different absorption intensities with respect to the biological substance. Therefore, a scattered light beam of one of the illumination light beams includes the data of the biological substance more abundantly than a scattered light beam of the other one of the illumination light beams. Accordingly, by performing the comparison operation processing between these two images with the image processing section, it becomes possible to enhance the data of the biological substance, and thereby the biological substance lying deep inside the biological tissue can be clearly visualized.

Moreover, the present invention also provides a biological observation apparatus comprising: a light source unit for emitting an illumination light beam including two kinds of wavelengths having different absorption intensities with respect to a biological substance; an irradiation optical system for irradiating a subject with the illumination light beam from the light source unit; a spectral optical system for dividing a scattered light beam into scattered light beams of the two kinds of wavelengths, when the subject is irradiated with the illumination light beam by the irradiation optical system; a detection optical system for respectively detecting the scattered light beams of the two kinds of wavelengths that have been divided by the spectral optical system, and acquiring images thereof; and an image processing section for performing comparison operation processing on the two images acquired by the detection optical system.

According to the present invention, an illumination light beam including two kinds of wavelengths is emitted by the operation of the light source unit, and is irradiated on the subject by the irradiation optical system. The thus irradiated illumination light beam is scattered by the subject, and is divided into scattered light beams of two kinds of wavelengths by the spectral optical system. Then, the thus divided scattered light beams of two kinds of wavelengths are respectively detected by the detection optical system, by which the respective images of these light beams are acquired. These two kinds of wavelengths included in the illumination light beam have different absorption intensities with respect to the biological substance. Therefore, a scattered light beam of one kind of wavelength includes the data of the biological substance more abundantly than a scattered light beam of the other kind of wavelength. Accordingly, by performing the comparison operation processing between these two images with the image processing section, it becomes possible to enhance the data of the biological substance, and thereby the biological substance lying deep inside the biological tissue can be clearly visualized.

In this invention, it is preferable that the two kinds of wavelengths are light wavelengths which have equivalent absorption intensities with respect to the biological tissue.
By so doing, it becomes possible to create an image in which the data of the biological tissue has been removed or sufficiently reduced, by the comparison operation processing, and the running pattern of blood vessels can be more clearly visualized.

Moreover, in this invention, the comparison operation processing may be a subtraction operation which subtracts one of the images from the other one of the images.
If the two kinds of wavelengths have equivalent absorption intensities with respect to the biological tissue, the data of the biological tissue can be removed by the subtraction operation.

Furthermore, in this invention, the comparison operation processing may be a division operation which divides one of the images by the other one of the images.
If the two kinds of wavelengths have equivalent absorption intensities for the biological tissue, it becomes possible by the division operation to acquire an image in which the data of the biological tissue is reduced and another biological substance buried in the biological tissue is enhanced.

Moreover, in this invention, the two kinds of wavelengths may be equal to or between 400 nm and 2300 nm. Furthermore, in this invention, one of the wavelengths may be equal to or between 400 nm and 1100 nm or equal to or between 1400 nm and 2300 nm, and the other one of the wavelengths may be equal to or between 600 nm and 850 nm or equal to or between 1000 nm and 1400 nm.
By so doing, it becomes possible to set the absorption coefficient of a substance contained in blood, for example a hemoglobin, to be 1 cm⁻¹ for the illumination light beam of one of the wavelengths and 5 cm⁻¹ for the illumination light beam of the other one of the wavelengths. Therefore, the running pattern of a blood vessel buried in the biological tissue, in particular a fat tissue, can be accurately visualized.

Moreover, in this invention, the one of the wavelengths may be equal to or between 400 nm and 600 nm or equal to or between 900 nm and 1100 nm.
By so doing, it becomes possible to select a wavelength for which the absorption coefficient of a hemoglobin or water comes to a peak, as one of the wavelengths.
Furthermore, the present invention provides an endoscopic apparatus comprising any one of the above-mentioned biological observation apparatus.

The present invention offers an effect in which a blood vessel lying deep inside a biological tissue can be detected more clearly despite the existence of the biological tissue, particularly, a fat tissue, with reference to a desired biological substance from the data of a deep area inside an organism's body.

### Brief Description of Drawings

[FIG. 1A] FIG. 1A is an overall schematic block diagram showing a biological observation apparatus and an endoscopic apparatus according to a first embodiment of the present invention.
[FIG. 1B] FIG. 1B is a front view of a filter turret of the endoscopic apparatus and the biological observation apparatus according to the first embodiment of the present invention.
[FIG. 2A] FIG. 2A shows an image of a scattered light beam acquired when an illumination light beam of a first wavelength is irradiated by the endoscopic apparatus of FIG. 1A.
[FIG. 2B] FIG. 2B shows the optical path when the illumination light beam of the first wavelength is irradiated by the endoscopic apparatus of FIG. 1A.
[FIG. 3A] FIG. 3A shows an image of a scattered light beam acquired when an illumination light beam of a second wavelength is irradiated by the endoscopic apparatus of FIG. 1A.
[FIG. 3B] FIG. 3B shows the optical path when the illumination light beam of the second wavelength is irradiated by the endoscopic apparatus of FIG. 1A.
[FIG. 4] FIG. 4 shows an example of an image created by comparison operation processing with the image processing section of the endoscopic apparatus of FIG. 1A.
[FIG. 5] FIG. 5 is an overall schematic block diagram showing a first modified example of the biological observation apparatus and the endoscopic apparatus of FIG. 1A.
[FIG. 6] FIG. 6 is an overall schematic block diagram showing a second modified example of the biological observation apparatus and the endoscopic apparatus of FIG. 1A.
[FIG. 7] FIG. 7 is an overall schematic block diagram showing a biological observation apparatus and an endoscopic apparatus according to a second embodiment of the present invention.
[FIG. 8] FIG. 8 is an overall schematic block diagram showing a modified example of the biological observation apparatus and the endoscopic apparatus of FIG. 7.

### Explanation of Reference:

- A:: Biological tissue (subject)
- λ1 and λ2:: Wavelength
- G1 and G2:: Image
- L1 and L2:: Illumination light beam
- S1 and S2:: Scattered light beam
- 1:: Biological observation apparatus
- 2:: Endoscopic apparatus
- 4:: Light source device (light source unit)
- 7:: Light guide (irradiation optical system)
- 8:: Object lens (detection optical system)
- 9:: Image guide (detection optical system)
- 12, 12a, and 12b:: Image-pickup section (detection optical system)
- 14:: Image processing section
- 16:: Spectral section (spectral optical system, light source switchover circuit)

### Best Mode for Carrying Out the Invention

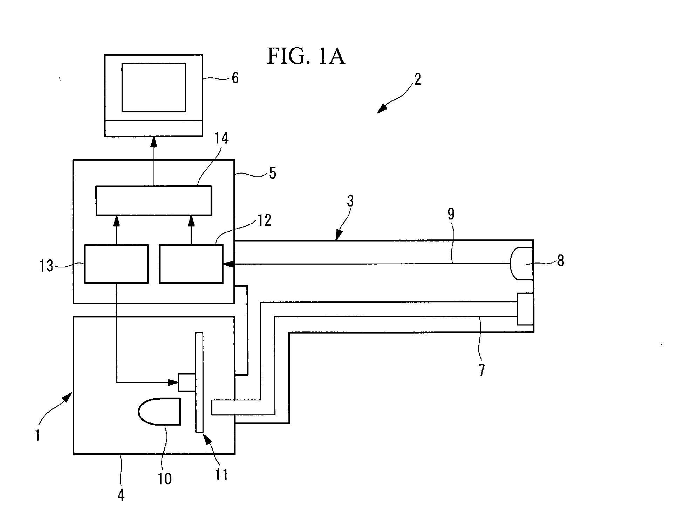

Hereunder is a description of a biological observation apparatus 1 and an endoscopic apparatus 2 according to a first embodiment of the present invention, with reference to FIG. 1A through FIG. 4.
The biological observation apparatus 1 of this embodiment is equipped in the endoscopic apparatus 2 which has a structure for visualizing the running pattern of blood vessels in a fat tissue as a biological tissue.

As shown in FIG. 1A, the endoscopic apparatus 2 comprises: a long and slender insertion portion 3 to be inserted in a body cavity; a light source device (light source unit) 4 placed on the proximal side of the insertion portion 3; a camera control unit (CCU) 5 for detecting a light beam which has been condensed at the distal end of the insertion portion 3, making an image of it, and applying an image processing thereto; and a monitor 6 for displaying the image that has been image-processed by the CCU 5.

The insertion portion 3 comprises: a light guide (irradiation optical system) 7 for guiding a light beam emitting from the light source device 4, along the longitudinal direction from the proximal side to the distal end of the insertion portion 3, to thereby irradiate an inner surface of a body cavity with the light beam; an object lens 8 for condensing a scattered light beam which has traveled from the inner surface of the body cavity into the inside thereof, then has been scattered by the biological tissue and the like, and now is returning to the inner surface of the body cavity; and an image guide 9 for guiding the scattered light beam that has been condensed by the object lens 8.

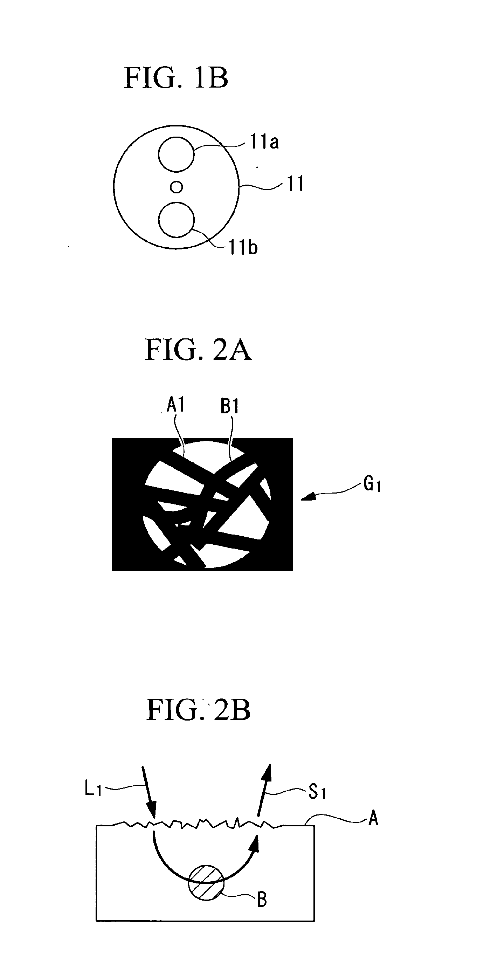

The light source device 4 comprises: a light source 10 such as a xenon lamp, a halogen lamp, a white LED, and a near-infrared LED, for generating a light beam of a relatively broad wavelength band including two kinds of wavelengths; and a filter turret 11 as a wavelength-switchover means, for selectively taking out light beams having two kinds of wavelengths from the light beam which has been emitted from the light source 10. As shown in FIG. 1B, the filter turret 11 comprises two filters 11a and 11b that respectively allow the transmissions of light beams having two kinds of wavelengths, for example, a first wavelength λ1 (400 nm ≤ λ1 ≤ 600 nm) and a second wavelength λ2 (1000 nm ≤ λ2 ≤ 1400 nm), corresponding to the biological substance. Here, as for the wavelength-selection means other than the filter turret 11, it is possible to use a spectral element which diffracts a light beam by grating, or it is also possible to execute video-rate imaging by a driven-type spectral device that is capable of a high-speed switchover operation between wavelengths (for example, a Fabry-Perot type wavelength variable element and a piezo-driven etalon spectral element).

The CCU 5 comprises: an image-pickup section 12 such as a CCD for capturing the scattered light beam that has been guided through the image guide 9; a control circuit 13 for associating the timing for switching between the respective filters 11a and 11b of the filter turret 11, with the image acquired by the image-pickup section 12; and an image processing section 14 for processing the acquired image.
The image processing section 14 creates a new image by subtracting the image acquired when the illumination light beam of the second wavelength λ2 is irradiated from the image acquired when the illumination light beam of the first wavelength λ1 is irradiated (comparison operation processing).

The biological observation apparatus 1 of this embodiment comprises the light source device 4, the light guide 7, the object lens 8, the image guide 9, the image-pickup section 12, the control circuit 13, and the image processing section 14.
Here is a description of the operation of the thus configured biological observation apparatus 1 and the thus configured endoscopic apparatus 2 according to this embodiment.

For the observation of an inner wall of a body cavity with the endoscopic apparatus 2 of this embodiment, the light source 10 of the light source device 4 is turned on to emit an illumination light beam, and the control circuit 13 controls the filter turret 11 to rotate so that the different filters 11a and 11b can be alternately placed on the optical axis of the illumination light beam. By so doing, the illumination light beam of the first wavelength λ1 and the illumination light beam of the second wavelength λ2 are alternately emitted from the light source device 4. Preferably, the control circuit 13 controls the filters 11a and 11b to pass by or stop at the optical axis of the illumination light beam, so that they can be repeatedly placed on the axis, so as to thereby carry out the observation by each wavelength in a timewise manner according to the movement inside the organism's body.

The two kinds of the illumination light beams alternately emitting from the light source device 4 are respectively guided through the light guide 7 of the insertion portion 3 to the distal end thereof, and then emit from the distal end of the insertion portion 3 toward the inner wall of the body cavity.
The illumination light beam L1 of the first wavelength λ1 has a wavelength whose absorption intensity comes to a peak with respect to a hemoglobin or water. Accordingly, as shown in FIG. 2B, out of the illumination light of the first wavelength λ1 which enters the biological tissue A from the inner wall of the body cavity, a light beam passing through a blood vessel B that lies deep inside the biological tissue A, is absorbed by blood and thus is scattered. Therefore, as shown in FIG. 2A, the scattered light beam S1 which is again emitting from the surface of the biological tissue A includes both the data B1 of the blood vessel B and the data A1 of the surface profile of the biological tissue A.

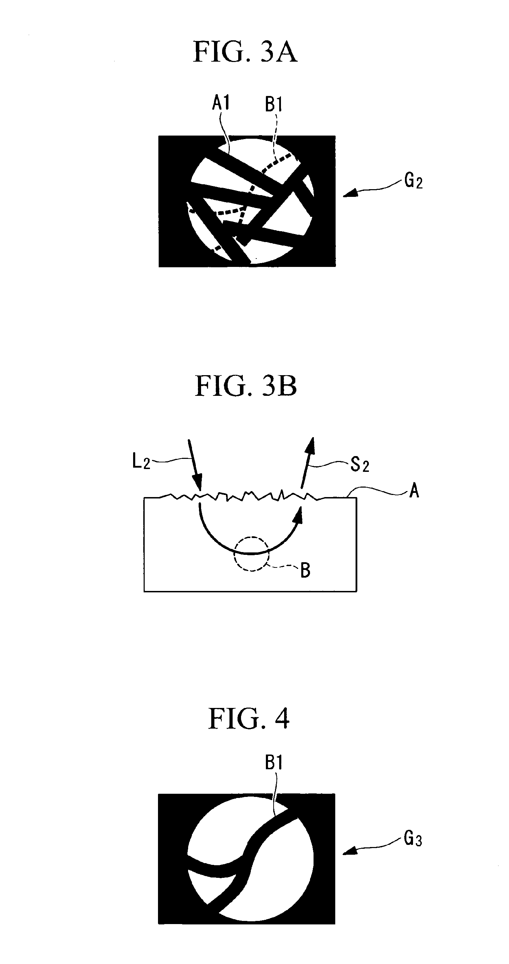

On the other hand, as shown in FIG. 3B, the illumination light beam L2 of the second wavelength λ2 has a small absorption coefficient with respect to a hemoglobin or water, which is equivalent to the absorption coefficient with respect to the biological tissue A, particularly, a fat tissue. Therefore, as shown in FIG. 3A, the scattered light beam S2 which has once entered from the inner wall of the body cavity into the biological tissue A and is again emitting from the surface of the biological tissue A does not include the data B1 of the blood vessel B (broken line) but abundantly includes the data A1 of the surface profile of the biological tissue A.

Then, the scattered light beams S1 and S2 resulting from the irradiation of these two kinds of illumination light beams L1 and L2 are respectively captured and made into images by the image-pickup section 12, and thereafter the respective images G1 and G2 are subjected to a classification regarding which kind of the illumination light beam L1 or L2 made the image G1 or G2, by the operation of the control circuit 13. In the image processing section 14, the image G1 resulting from the irradiation of the illumination light beam L1 of the first wavelength λ1 is subtracted by the image G2 resulting from the irradiation of the illumination light beam L2 of the second wavelength λ2. In the thus obtained differential image G3, as shown in FIG. 4, only the image of the data B1 of the blood vessel B appears as the difference, which is to be displayed on the monitor 6.

In this manner, the comparison operation is performed between images captured by different kinds of wavelengths (two kinds in this example), one of which can make a significant difference in the absorption coefficient of at least 1 cm⁻¹ or greater (4 cm⁻¹ in this example) between a main component (hemoglobin and water in this example) of a biological substance (blood or blood vessel in this example) and a biological tissue (fat tissue in this example), and the other one of which has equivalent absorption coefficients with respect to both the biological substance and the biological tissue. By so doing, it becomes possible to create an image in which the data of the biological tissue has been removed or sufficiently reduced. Accordingly, the biological observation apparatus 1 and the endoscopic apparatus 2 of this embodiment are advantageously capable of visualizing the blood vessel B which lies deep inside the biological tissue A more clearly despite the existence of the biological tissue A, particularly, a fat tissue.
Once the running pattern of the blood vessel B which lies deep inside the biological tissue A is visualized, the risk of injuring the blood vessel B can be readily avoided at the time for incising a human body, and therefore the operation time can be shortened.

In particular, since the light wavelength-dependent characteristic of the biological tissue A is utilized to visualize the running pattern of the blood vessel B, this method is noninvasive and capable of alleviating the burden on the patient, and enables an inexpensive observation without using CT and MRI.

In this embodiment, a wavelength meeting a range of 400 nm ≤ λ1 ≤ 600 nm is selected for the first wavelength λ1, and a wavelength meeting a range of 1000 nm ≤ λ2 ≤ 1400 nm is selected for the second wavelength λ2. However, the wavelengths are not limited to these ranges. For example, it is also possible to use a wavelength meeting a range of 400 nm ≤ λ1 ≤ 1100 or 1400 nm ≤ λ1 ≤ 2300 nm as the first wavelength λ1, and to use a wavelength meeting a range of 600 nm ≤ λ2 ≤ 850 nm or 1000 nm ≤ λ2 ≤ 1400 nm as the second wavelength λ2.

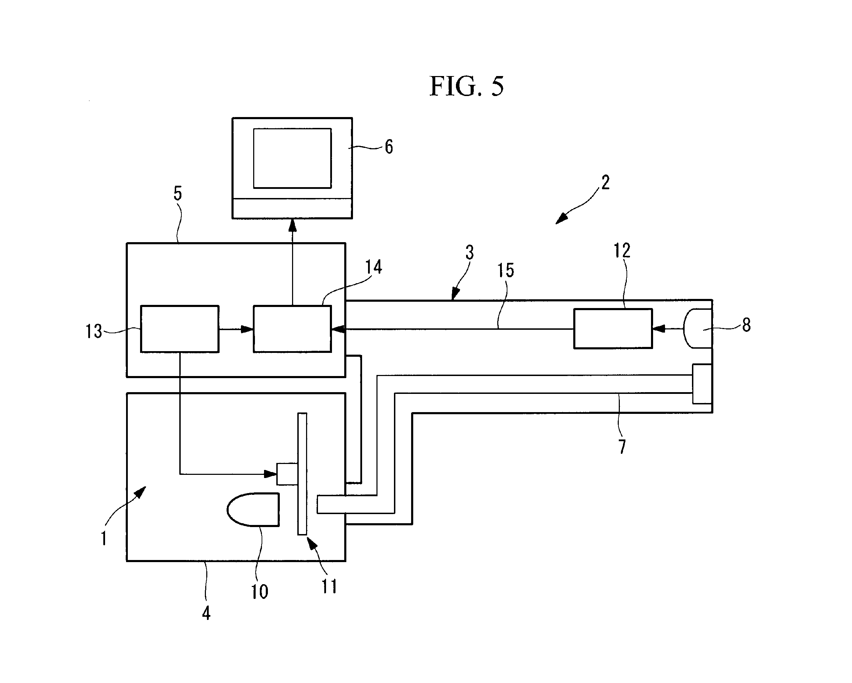

In addition, in this embodiment, the image-pickup section 12 such as a CCD is set in the CCU 5 that is provided on the proximal side of the insertion portion 3. However, instead of this arrangement, it is also possible as shown in FIG. 5 to set the image-pickup section 12 on the distal end of the insertion portion 3 so that an electric signal converted from the image data can be transmitted to the CCU 5 through a wire 15.

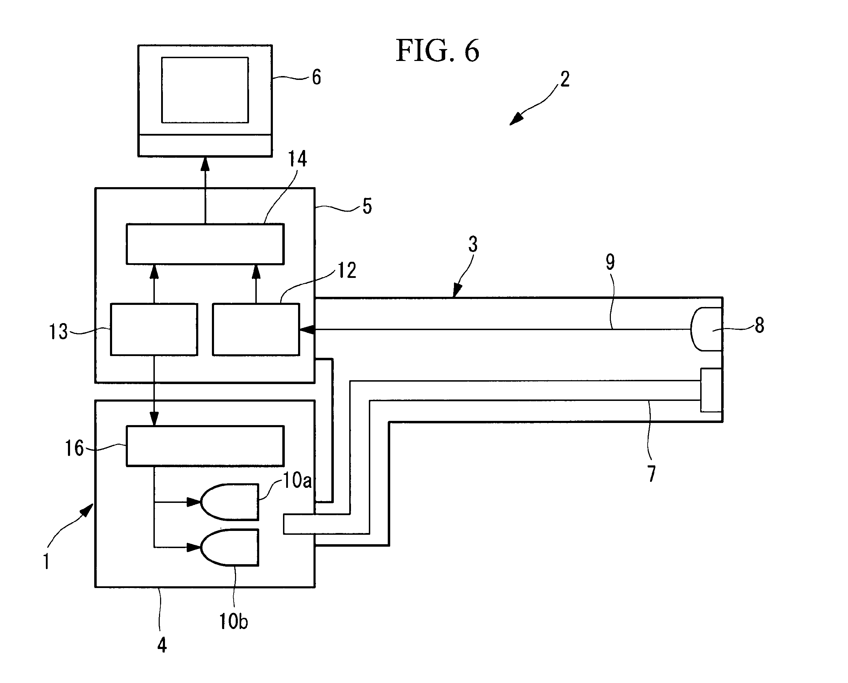

Moreover, in this embodiment, the wavelengths λ1 and λ2 of the illumination light beams L1 and L2 emitting from the light source device 4 are switched over by the rotation of the filter turret 11. However, instead of this configuration, it is also possible as shown in FIG. 6 to separately prepare light sources 10a and 10b whose center wavelengths are respectively the first wavelength λ1 and the second wavelength λ2 so that the lighting of the light source 10a of the first wavelength λ1 and the lighting of the light source 10b of the second wavelength λ2 can be alternately switched over according to the command signal from a light source switchover circuit 16.

Furthermore, in this embodiment, the subtraction operation between two images G1 and G2 is performed as the comparison operation processing in the image processing section 14. However, instead of this operation, it is also possible to perform a division operation. This can also create the image G3 that enhances the blood vessel B.

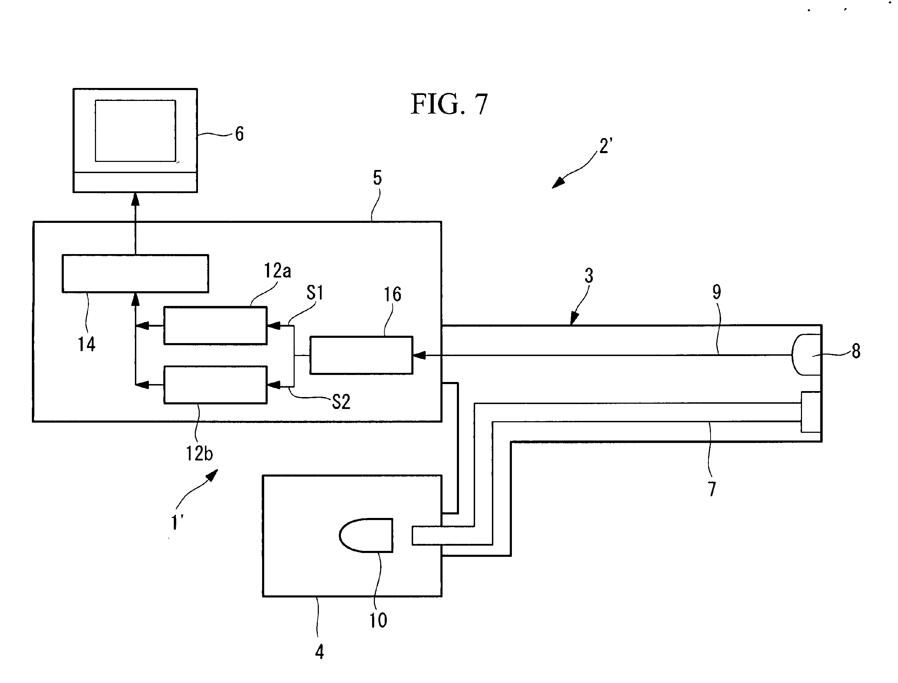

Next, hereunder is a description of a biological observation apparatus 1' and an endoscopic apparatus 2' according to a second embodiment of the present invention, with reference to FIG. 7.
In the description of this embodiment, parts sharing common structures with those of the biological observation apparatus 1 and the endoscopic apparatus 2 of the above-mentioned first embodiment are denoted by the same reference symbols, and are not explained herein.

The biological observation apparatus 1' of this embodiment is different from the biological observation apparatus 1 of the first embodiment, in the point where the filter turret 11 is not provided in the light source device 4, but instead a spectral section 16 for dividing the scattered light beam that has been guided through the image guide 9 into two beams is provided in the CCU 5. In addition, the biological observation apparatus 1' of this embodiment is different from the biological observation apparatus 1 of the first embodiment, also in the point where two image-pickup sections 12a and 12b are provided so as to respectively capture the thus divided two scattered light beams.

Examples of the spectral section 16 can include a dichroic mirror, a diffraction grating, and a prism. The spectral section 16 is designed to respectively extract the scattered light beams S1 and S2 having the wavelengths λ1 and λ2 by allowing the transmission of the scattered light beam that has been guided through the image guide 9. Then, the scattered light beam S1 of the first wavelength λ1 is captured by the first image-pickup section 12a, and the scattered light beam S2 of the second wavelength λ2 is captured by the second image-pickup section 12b.

The scattered light beam S1 of the first wavelength λ1 extracted from the scattered light beam includes both the data B1 of the blood vessel B and the data A1 of the surface profile of the biological tissue A, while the scattered light beam S2 of the second wavelength λ2 extracted from the scattered light beam includes only the data A1 of the surface profile of the biological tissue A.
Accordingly, by performing the subtraction operation between the images G1 and G2 which have been respectively acquired by capturing the thus divided scattered light beams S1 and S2, it becomes possible to create the image G3 in which the data A1 of the surface profile of the biological tissue A has been removed and the data B1 of the blood vessel B clearly comes out.

In this embodiment, the spectral section 16 and the two image-pickup sections 12a and 12b are set in the CCU 5. However, instead of this setting, it is also possible to set them at the distal end of the insertion portion 3. In this case, electric signals output from the respective image-pickup sections 12a and 12b may be transmitted to the image processing section 14 of the CCU 5.

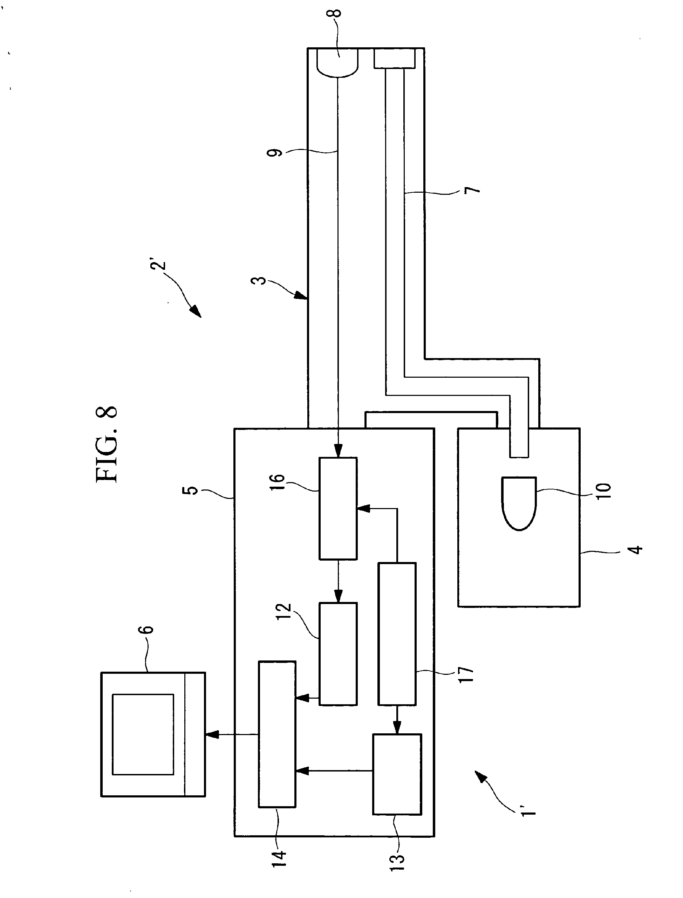

In addition, in this embodiment, as for the spectral section 16, it is also possible to employ a device as shown in FIG. 8 which can switch over the wavelength of the scattered light beam passing therethrough according to the command signal from a spectral switchover section 17. By so doing, only one image-pickup section 12 is needed, and therefore the size and the cost can be much more reduced. In this case, the control circuit 13 can be used for associating the switchover timing for the spectral switchover section 17 with the acquired image G1 or G2.

The present invention is not to be limited to the above-mentioned embodiments, and various modifications can be made on the basis of the aforementioned gist. For example, the biological substance serving as the observation target of the present invention is not limited to blood which can be indicated by red blood corpuscles that mainly consist of hemoglobins, but may be any other biological substance having a distinct light absorption characteristic, such as a blood vessel wall, a lymphatic vessel, a lymphatic fluid, a lymph node, a nerve cell, a tumor cell, a collagen, and a melanin. In addition, the biological tissue which intervenes between the biological substance and the optical path for the observation of the present invention can also be exemplified by a fat tissue, a subcutaneous tissue, a bone tissue, and a muscular tissue. Occasionally, in order to observe a fat component which can be generated in a subcutaneous tissue of any organ, this fat component (which is not a biological tissue) may be referred to as a biological substance. After all, the present invention can be applicable because the observation of any substance having a distinct light absorptivity in an organism's body is enabled by minimizing the influence of another intervening substance. In addition, it is preferable to modify the wavelength range of the illumination light beam to be used, according to the target biological substance. Moreover, it is also possible to modify the wavelength of the illumination light beam to be used, according to other biological substances. If three or more types of biological substances are mixedly present or overlaid deep inside an organism's body, it is also possible to alternately irradiate light beams having three kinds of wavelengths or more to perform subtraction or division operations on the respective images thereof. For example, if a fat tissue lies deep inside a subcutaneous tissue, it is preferable to select at least two kinds of light wavelengths having equivalent absorption intensities with respect to both the subcutaneous tissue and the fat tissue so that various biological substances lying within or deep inside the fat tissue can be observed.

Moreover, in the image processing section, instead of displaying the running pattern of a blood vessel that has been made by the subtraction or division operation of two images, if, conversely, an image of the fat tissue only in which the blood vessel-related data has been completely removed or an image which enhances both the blood vessel and the fat tissue is displayed on the monitor, it is also possible to help endoscopic treatments (for example, fat resection). Furthermore, real time observation of an image which follows the movement of the organism's body per se or the biological substance, or an image which reflects the latest concentration or density of the biological substance, is also possible by acquiring image-processed images in a timewise manner and displaying them on the monitor. In addition, the applicable endoscope of the present invention is not limited to the above-mentioned embodiments, and may be various types of endoscopes (such as a rigid endoscope, a flexible endoscope, an endoscope for laparoscopic surgery, and a capsule endoscope), and a device or a system being an ensemble of a treatment instrument for surgical procedures such as grasping, resection, and suturing, and an endoscope which has another observation means such as fluorescence, polarized light, or ultrasonic waves.

## Claims

1. A biological observation apparatus comprising:
a light source unit capable of selectively emitting illumination light beams of two kinds of wavelengths having different absorption intensities with respect to a biological substance;
an irradiation optical system for irradiating a subject with the illumination light beams from the light source unit;
a detection optical system for detecting scattered light beams when the subject is irradiated with the illumination light beams of said two kinds of wavelengths by the irradiation optical system, and acquiring respective images thereof; and
an image processing section for performing comparison operation processing on the two images acquired by the detection optical system.

2. A biological observation apparatus comprising:
a light source unit for emitting an illumination light beam including two kinds of wavelengths having different absorption intensities with respect to a biological substance;
an irradiation optical system for irradiating a subject with the illumination light beam from the light source unit;
a spectral optical system for dividing a scattered light beam into scattered light beams of said two kinds of wavelengths, when the subject is irradiated with the illumination light beam by the irradiation optical system;
a detection optical system for respectively detecting the scattered light beams of the two kinds of wavelengths that have been divided by the spectral optical system, and acquiring images thereof; and
an image processing section for performing comparison operation processing on the two images acquired by the detection optical system.

3. A biological observation apparatus according to either one of claim 1 and claim 2, wherein said two kinds of wavelengths are light wavelengths which have equivalent absorption intensities with respect to the biological tissue.

4. A biological observation apparatus according to claim 3, wherein said comparison operation processing is a subtraction operation which subtracts one of the images from the other one of the images.

5. A biological observation apparatus according to claim 3, wherein said comparison operation processing is a division operation which divides one of the images by the other one of the images.

6. A biological observation apparatus according to any one of claim 1 through claim 5, wherein said two kinds of wavelengths are equal to or between 400 nm and 2300 nm.

7. A biological observation apparatus according to claim 6, wherein one of the wavelengths is equal to or between 400 nm and 1100 nm or equal to or between 1400 nm and 2300 nm, and the other one of the wavelengths is equal to or between 600 nm and 850 nm or equal to or between 1000 nm and 1400 nm.

8. A biological observation apparatus according to claim 7, wherein said one of the wavelengths is equal to or between 400 nm and 600 nm or equal to or between 900 nm and 1100 nm.

9. An endoscopic apparatus comprising the biological observation apparatus according to any one of claim 1 through claim 8.
